(19) **Europäisches Patentamt / European Patent Office / Office européen des brevets**

(11) **EP 3 336 173 A1**

(12) # EUROPEAN PATENT APPLICATION

(43) Date of publication:
**20.06.2018 Bulletin 2018/25**

(21) Application number: **16382621.7**

(22) Date of filing: **19.12.2016**

(51) Int Cl.:
*C12N 1/14* [(2006.01)]  *C12P 1/02* [(2006.01)]
*A61K 8/99* [(2017.01)]  *A61Q 19/00* [(2006.01)]
*A61Q 19/08* [(2006.01)]  *C12R 1/66* [(2006.01)]

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Infinitec Activos S.L.**
  **08170 Montornés del Vallès, Barcelona (ES)**
• **Instituto Biomar S.A.**
  **24009 Armunia, León (ES)**

(72) Inventors:
• **VINUESA NAVARRO, María De Los Ángeles**
  **E-24228 Valdefresno de la Sobarriba-León (ES)**
• **CALVO PELAZ, Belinda**
  **E-24002 León (ES)**
• **MOURELLE MANCINI, Marisabel**
  **E-08028 Barcelona (ES)**

(74) Representative: **ABG Patentes, S.L.**
  **Avenida de Burgos, 16D**
  **Edificio Euromor**
  **28036 Madrid (ES)**

(54) **A STRAIN OF ASPERGILLUS VERSICOLOR AND APPLICATIONS THEREOF**

(57) The invention relates to a strain of Aspergillus versicolor and to a biologically pure culture thereof. The invention also relates to a broth obtainable by aerobic incubation of a carbon source with said microorganism and to an extract of said broth.

EP 3 336 173 A1

**Description**

FIELD OF THE INVENTION

The invention relates to a strain of *Aspergillus versicolor* and to a biologically pure culture thereof. The invention also relates to a broth obtainable by aerobic incubation of a carbon source with said microorganism and to an extract of said broth.

BACKGROUND OF THE INVENTION

The skin constitutes a physical barrier between an organism and their environment. The skin is composed of two tissues: the epidermis and the dermis. The dermis forms approximately 90% of the thickness of the skin, containing collagen, elastin, several differentiated structures such as blood vessels, sweat glands, and mainly cell-types such as fibroblasts, macrophages and adipocytes.

Skin aging is a biological process that induces changes in the structural integrity and physiological function of the skin. This process is characterized by the progressive loss of structural integrity and physiological changes caused by intrinsic and extrinsic determinants lead to aging and degradation of biological functions, due to the inability of the organism to adapt to the metabolic stress over time.

Exposure to UV radiation is one of the most significant factors inducing stress, and the major cause of premature skin aging. The formation of wrinkles is an important consequence of intrinsic aging and photodamage, both associated with oxidative stress and inflammatory response.

Oxidative stress occurs when oxidant bioactive products such as free radicals and reactive oxygen species (ROS) which reduce defense activity of endogenous antioxidant systems. ROS are generated in all cells during the course of normal metabolic processes, for example in the respiratory chain, and low levels of ROS are required for cell signaling. However, high levels of these aggressive molecules may attack other cellular macromolecules, which may result in severe cellular damage. This process often begins with an inflammatory response, producing a potential damage cellular organelles, which can be associated with skin conditions.

These "free radicals" are removed or inactivated in vivo by endogenous and exogenous antioxidants. A relative or absolute deficiency of endogenous antioxidant defenses can lead to a situation of oxidative stress with accumulation of free radicals that can cause a large number of harmful effects on the skin by activating enzymes, such as tyrosinase and elastase, contributing to skin aging.

Surprisingly, the applicant of this invention has found a strain of *Aspergillus versicolor* which is capable of producing an aerobic incubation broth which, upon extraction with organic solvents and removal of said solvents, yields an extract which with anti-oxidant capacity which has very interesting cosmetic properties such as a) protecting the skin against oxidative stress, b) reducing the presence of advanced glycation end-products, c) increasing the synthesis of intracellular ATP, d) increase skin density, e) increase skin thickness, f) protecting the skin from the effects of contamination, g) protecting the skin from the effects UVb radiation.

SUMMARY OF THE INVENTION

The invention provides a an extract obtainable by an aerobic incubation process using the exopolysaccharide excreted by a strain of *Aspergillus versicolor* which extract is susceptible of being used to improve skin condition.

DESCRIPTION OF THE INVENTION

This invention relates to a strain of *Aspergillus versicolor* and to a biologically pure culture thereof. The invention also relates to a broth obtainable by aerobic incubation of a carbon source with said microorganism and to an extract of said broth which extract is susceptible of being used for the treatment and/or care of the skin.

Definitions

In order to facilitate the comprehension of this invention, the meanings of some terms and expressions as used in the context of the invention are included.

In the context of this invention "skin" is understood to be the layers which comprise it, from the uppermost layer or stratum corneum to the lowermost layer or hypodermis, both inclusive. These layers are composed of different types of cells such as keratinocytes, fibroblasts, melanocytes and/or adipocytes among others. In the context of this invention, the term "skin" includes the scalp.

The term "cosmetic, non-therapeutic treatment" refers to the application of a product of the invention to the skin in particular with the aim of improving the cosmetic qualities of the skin such as and not restricted to, their level of hydration, elasticity, firmness, shine, tone or texture, among others. The term "care" in this invention refers to the maintenance of the qualities of the skin and it includes the body and/or hair hygiene. These qualities are subject to improvement and maintained through a cosmetic treatment and/or care of the skin in healthy subjects.

The term "prevention", as used in this invention, refers to the ability of a product of the invention to prevent, delay or hinder the appearance or development of an aging sign before its appearance.

In the context of this invention, the term "aging" refers to the changes experienced by the skin with age (chronoaging) or through exposure to the sun (photoaging) or to environmental agents such as tobacco smoke, extreme climatic conditions of cold, heat, or wind, chemical contaminants or pollutants, and includes all the external visible and/or perceptible changes through touch, such as and not restricted to, the development of discontinuities on the skin such as wrinkles, fine lines, furrows, irregularities or roughness, increase in the size of pores, loss of elasticity, loss of firmness, loss of smoothness, loss of the capacity to recover from deformation, sagging of the skin such as sagging cheeks, the appearance of bags under the eyes or the appearance of a double chin, among others, changes to the color of the skin such as spots, reddening, bags under the eyes or the appearance of hyper-pigmented areas such as age spots or freckles among others, anomalous differentiation, hyper-keratinization, elastosis, keratosis, hair loss, orange-peel skin, loss of collagen structure and other histological changes of the stratum corneum, hypodermis, dermis, epidermis, vascular system (for example the appearance of spider veins or telangiectasias) or of those tissues close to the skin, among others. The term "photoaging" groups together the set of processes due to the prolonged exposure of the skin to ultraviolet radiation which result in the premature aging of the skin, and presents the same physical characteristics as aging, such as and not restricted to, flaccidity, sagging, changes to the color or irregularities in the pigmentation, abnormal and/or excessive keratinization.

The term "broth", as used in this invention, refers to the liquid composition comprising a microorganism from the species *Aspergillus versicolor* deposited in the Spanish Type Culture Collection (CECT) with accession number CECT 20952 which results from allowing said microorganism to grow in the presence of an aqueous composition having a pH between 5 and 7 and comprising a source of carbon, a source of $K^+$, a source of $Mg^{2+}$, a source of $Na^+$, a source of $Cl^-$, a source of $SO_4^{2-}$, a source of $PO_4^{3-}$.

The term "extract", as used in this invention, refers to a product obtainable by extracting with an organic solvent such as ethyl acetate or a mixture of ethyl acetate and methanol from the broth according to the invention, separating the solvent layer and subsequently removing the solvent from said layer.

A first aspect of the present invention relates to a microorganism from the species *Aspergillus versicolor* deposited in the Spanish Type Culture Collection (CECT) with accession number CECT 20952.

A second aspect of the present invention relates to a biologically pure culture of a microorganism from the species *Aspergillus versicolor* deposited in the Spanish Type Culture Collection (CECT) with accession number CECT 20952.

A third aspect of the present invention relates to a broth obtainable by incubating an aqueous composition comprising a source of carbon, a source of $K^+$, a source of $Mg^{2+}$, a source of $Na^+$, a source of $Cl^-$, a source of $SO_4^{2-}$, a source of $PO_4^{3-}$ in the presence of a microorganism from the species *Aspergillus versicolor* deposited in the Spanish Type Culture Collection (CECT) with accession number CECT 20952 at a pH between 6 and 7.

A fourth aspect of the present invention relates to an extract obtainable by separating the microorganism from the broth according to the third aspect of the present invention, extracting the resulting solution with an organic solvent such as ethyl acetate, recovering the organic layer and removing the organic solvent from said organic layer.

Another aspect which does not form part of the present invention relates to a cosmetic composition comprising an effective cosmetic quantity of the extract as defined in the fourth aspect of the invention and at least one cosmetically acceptable excipient, adjuvant and/or ingredient.

Another aspect which does not form part of the present invention relates to the use of the extract of the fourth aspect for the cosmetic, non-therapeutic treatment and/or care of the skin.

Another aspect which does not form part of the present invention relates to the use of the cosmetic composition of the fifth aspect for the cosmetic, non-therapeutic treatment and/or care of the skin.

Another aspect which does not form part of the present invention relates to the use of the extract of the fourth aspect for the manufacture of a cosmetic composition for treating the skin.

Preferably, the cosmetic, non-therapeutic treatment and/or care of the skin is capable of one or more of the following actions: a) reducing the presence of advanced glycation end-products, b) protecting the skin against oxidative stress, c) increasing the synthesis of intracellular ATP, d) increase skin density, e) increase skin thickness, f) protecting the skin from the effects of contamination, g) protecting the skin from the effects UVb radiation.

It is also disclosed that the treatment and/or care of the skin is carried out by topical or transdermal application.

*Aspergillus versicolor* is a strain of *Aspergillus versicolor* species with deposit number CECT 20952. Said strain has been deposited in the Colección Española de Cultivos Tipo (CECT) at the Universidad de Valencia, Spain as institution legally recognized for said purpose according to the Budapest Treaty on the International Recognition of the Deposit of Microorganisms on April 28, 1977. This deposit has been made under the provisions of the Budapest Treaty and all restrictions on the availability thereof to the public will be irrevocably maintained upon the granting of a patent on this application.

The organism was isolated from a sample collected at 1-3 m deep at Muelle de Colon, Isla de Colon, Panama in April 2000. The collection of the sample was in accordance to the Panamanian legislation about the access to the genetic resources in its territories. This sample is in compliance with the Nagoya protocol, with the EU rules and with the Panamanian legislation on Access and Benefit Sharing (ABS), and that, in conformity with those laws, they are free of charges in regards to ABS requirements.

In a particular embodiment, a broth can be obtained through aerobic incubation of the strain of *Aspergillus versicolor* in a suitable culture medium, conventionally stirred and aerated. Aerobic incubation can be carried out in an aqueous nutrient medium stirred and aerated at a temperature between 15°C and 30°C, preferably between 24 °C and 28 °C, the medium having a pH between 5.0 and 9.0, preferably between 5.0 and 7.0, adjusting it if necessary during aerobic incubation. The duration of the aerobic incubation is between 72 to 240 hours, preferably between 120 and 216 hours, more preferably for 168 hours.

In a particular embodiment, in the aerobic incubation of the strain of *Aspergillus versicolor* aqueous nutrient medium is used which comprises a source of carbon, a source of $K^+$, a source of $Mg^{2+}$, a source of $Na^+$, a source of $Cl^-$, a source of $SO_4^{2-}$ and a source of $PO_4^{3-}$. Exogenous sugars, such as and not restricted to, galactose, glucose, mannose, amygdalin, cellobiose, maltose, starch, glycogen, lactose, mixtures thereof and/or compositions containing mixtures of these sugars can be used as a carbon source. In particular, an exogenous supply of oat meal and/or malt extract at a concentration of 1-5% is provided.

In another particular embodiment sources of other ions such as $NH_4^+$, $Ca^{2+}$, and $CO_3^{2-}$ and/or sources of trace elements such as Cu, Mn, Fe and Zn may be provided in the aqueous nutrient medium.

In another particular embodiment, the method of obtaining and extract from the aerobic incubation broth involves the following steps: a) contacting the broth with a solvent or mixtures of solvents which is not fully soluble in water. Preferably the solvent is selected from ethyl acetate and mixtures of ethyl acetate and methanol.

It is also disclosed a cosmetic composition characterized in that it comprises a cosmetically effective quantity of the extract of the present invention and at least one cosmetically acceptable excipient and/or ingredient. Said compositions can be prepared by the conventional methods known by the persons skilled in the art ["Harry's Cosmeti-cology", Seventh edition, (1982), Wilkinson J.B., Moore R.J., ed. Longman House, Essex, GB].

The cosmetically effective quantity of the extract in the composition to be administered, as well as its dosage, will depend on numerous factors, including age, condition of the recipient, the nature or severity of the condition to be treated and/or cared for and the route and frequency of administration.

"Cosmetically effective quantity" is understood to be a non-toxic but sufficient quantity of an ingredient to provide the desired effect. In particular, the extract of the invention is used at cosmetic concentrations to achieve the desired effect; in a preferred form, with regard to the total weight of the composition, between 0.0000000001% (in weight) and 20% (in weight); preferably between 0.00000001% (in weight) and 10% (in weight), more preferably between 0.000001% (in weight) and 5% (in weight) and even more preferably between 0.0001% (in weight) and 5% (in weight).

It is also disclosed that the extract of the invention can also be incorporated into cosmetic delivery systems and/or sustained release systems.

The term "delivery systems" relates to a diluent, adjuvant, excipient, vehicle or additive with which the extract of the invention is administered. These cosmetic or pharmaceutical carriers can be liquids, such as water, oils or surfactants, including those of petroleum, animal, plant or synthetic origin, such as and not restricted to, peanut oil, soybean oil, mineral oil, sesame oil, castor oil, polysorbates, sorbitan esters, ether sulfates, sulfates, betaines, glycosides, maltosides, fatty alcohols, nonoxynols, poloxamers, polyoxyethylenes, polyethylene glycols, dextrose, glycerol, digitonin and similar. A person skilled in the art knows the diluents, adjuvants or excipients which can be used in the different delivery systems in which the compound of the invention can be administered.

The term "sustained release" is used in a conventional sense relating to a delivery system of a compound which provides the gradual release of this compound during a period of time and preferably, although not necessarily, with relatively constant compound release levels over a period of time.

Examples of delivery or sustained release systems include, without limiting sense, liposomes, mixed liposomes, oleosomes, niosomes, ethosomes, milliparticles, microparticles, polymeric nanoparticles and solid lipid nanoparticles, nanostructured lipid supports, sponges, cyclodextrins, vesicles, micelles, mixed micelles of surfactants, surfactant-phospholipid mixed micelles, millispheres, microspheres and nanospheres, lipospheres, millicapsules, microcapsules and nanocapsules, as well as microemulsions and nanoemulsions, which can be added to achieve a greater penetration of the active ingredient and/or to improve the pharmacokinetic and pharmacodynamic properties of it. Preferred delivery or sustained release systems are liposomes, surfactant-phospholipid mixed micelles and microemulsions, more preferably water-in-oil microemulsions with an internal reverse micelle structure and nanocapsules containing microemulsions.

The sustained release systems can be prepared by methods known in the prior art, and the compositions which contain them can be administered, for example, by topical or transdermal administration, including adhesive patches, non-adhesive patches, occlusive patches and microelectric patches, or by systemic administration, for example and not restricted to, oral or parenteral route, including nasal, rectal or subcutaneous implantation or injection, or direct implantation or injection into a specific body part, and preferably should release a relatively constant quantity of the compound of the invention. The amount of extract contained in the sustained release system will depend, for example, on where the composition is to be administered, the kinetics and duration of the release of the extract of the invention, as well as the nature of the condition, disorder and/or disease to be treated and/or cared for.

It is also disclosed that cosmetic compositions containing the extract of this invention can be used in different types of compositions of topical or transdermal application, optionally including cosmetically acceptable excipients necessary for formulating the desired administration form.

The compositions of topical or transdermal application can be produced in any solid, liquid or semi-solid formulation, such as and not restricted to, creams, multiple emulsions such as and not restricted to, oil and/or silicone in water emulsions, water-in-oil and/or silicone emulsions, water/oil/water or water/silicone/water type emulsions, and oil/water/oil or silicone/water/silicone type emulsions, liquid crystals, anhydrous compositions, aqueous dispersions, oils, milks, balsams, foams, lotions, gels, cream gels, hydroalcoholic solutions, hydroglycolic solutions, hydrogels, liniments, sera, soaps, shampoos, conditioners, serums, polysaccharide films, ointments, mousses, pomades, powders, bars, pencils and sprays or aerosols (sprays), including leave-on and rinse-off formulations. These topical or transdermal application formulations can be incorporated using techniques known by the person skilled in the art into different types of solid accessories such as and not restricted to, bandages, gauzes, t-shirts, socks, tights, underwear, girdles, gloves, diapers, sanitary napkins, dressings, bedspreads, wipes, adhesive patches, non-adhesive patches, occlusive patches, microelectric patches or face masks, or they can be incorporated into different make-up products such as make-up foundation, such as fluid foundations and compact foundations, make-up removal lotions, make-up removal milks, under-eye concealers, eye shadows, lipsticks, lip protectors, lip gloss and powders, among others.

## Applications

It is disclosed the use of the extract of the invention in the preparation of a cosmetic composition for the treatment and/or care of the skin. In particular, the treatment and/or care refers to the treatment and/or prevention of skin aging, treatment and/or prevention of skin wrinkles, treatment of skin firming and/or for prevention of loss of skin firmness and antipollution protective agent.

The frequency of the application or administration can vary widely, depending on the needs of each subject, suggesting a range of application or administration from once per month to 10 times per day, preferably from once per week to 4 times per day, more preferably from three times per week to three times per day, even more preferably once per day.

## DEPOSIT OF BIOLOGICAL MATERIAL

The strain of the *Aspergillus versicolor* species was deposited under the conditions of the Budapest Treaty at the Colección Española de Cultivos Tipo (CECT) (http://www.uv.es/cect) at the Universidad de Valencia with the following address: Edificio 3 CUE. Parc Cientific Universitat de Valencia, Catedrático Agustin Escardino, 9, 46980 Paterna (Valencia). The deposit was done on May 11th, 2016 and the deposit number was CECT 20952.

## EXAMPLES

## EXAMPLE 1: Taxonomic studies

Taxonomic studies of the *strain Aspergillus versicolor* are summarised as follows:

## Culture characteristics:

Colonies reach 4 cm diameter in 15 days at 25°C on potato dextrose agar supplemented with 50% tropical sea salt in a culture chamber that maintains a humidity of 42%.

## Colony characteristics:

Colonies growing moderately, cottony, white at the beginning, turning yellow-green after 10 days, when sporulation starts to occur.

Microscopy:

Hyaline vegetative hyphae. Conidiophores erect, hyaline, bearing an apical radiated head, typically produced by Aspergillus (metulae). Biseriated conidiogenous cells showing spherical conidia, lightly echinulated. Conidia 2-3 $\mu$m diameter.

Molecular taxonomy:

Taxonomical determination was achieved after a sequencing analysis of ITS1-5.8S-ITS2 ribosomal DNA region. A 100% match was obtained for *Aspergillus versicolor.*

Growth conditions:

The optimal temperature for growth on solid media is 24-28 °C. The pH range for growth is between 5 and 7. Growth was best with flour. Other carbon sources such as starch, glycerol, and dextrose can also be used.
In shake cultures, the mycelium grows in small and compacted pellets. No sporulation is observed in submerged conditions, although typical Aspergillus conidiophores appear.
Based on the preceding characteristics the culture has been determined as a member of the species *Aspergillus versicolor.*

EXAMPLE 2: Production of extract of *Aspergillus versicolor*

*Aspergillus versicolor* when cultured under controlled conditions in a suitable medium and extracted with a mixture of organic solvents, produces an extract showing antioxidant properties.
This strain is grown in an aqueous nutrient medium, under aerobic conditions, preferably between 24 and 28 °C at a pH 5.0 to 7.0.
A description of the process is as follows:

Stock culture. A pure culture of *Aspergillus versicolor* was kept frozen at -70°C in 20% glycerol.

Preparation of inoculum. Two plugs of 1cm diameter from a well grown agar culture was used to inoculate 10 ml of seed medium containing 2% oat meal, 2% malt extract, 0.01% $KH_2PO4$, 0.005% $MgSO_4$, 0.5% NaCl, 0.02% KCl 0.24% $MgCl_2.6H_2O$, 0.75% $Na_2SO_4$ and tap water in 50 ml shake flasks and cultured at 25 C on a rotary shaker at 200 rpm. Medium is adjusted to pH 6.7. The flasks were incubated 48 hours in the dark, and used as a first stage inoculum. A second stage inoculum is done in the same medium, in 250ml flasks containing 40ml medium and 8% stage 1 inoculum. It is incubated 48h in the same conditions.
Aerobic incubation. 250 ml of the same medium in 2L Erlenmeyer flasks were inoculated with 8% of the second stage inoculum. The aerobic incubation was carried for 7 days, in the dark at 25 °C on a rotary shaker at 200 rpm.
Extraction. 1L of washed wet resin (Amberlite® XAD-1180) was added to a volume of 10L culture broth of the microorganism *Aspergillus versicolor,* used as a polymeric adsorbent resin.
Prior to use, the adsorbent resin was soaked and swelled in 50% (v/v) methanol/water for 12 h to remove impurities and the solvent was removed by washing with distilled water sufficiently.
The culture broth with the resin was stirred for 1h, and then the culture sample containing mycelium and the resin was separated from the supernatant by filtration through Radifil® (diatomaceous earth), and extracted with 5L of a solvent mixture (EtOAc/MeOH 3:1) for 1h. After that, the resultant suspension was filtered and partitioned between EtOAc and water. The organic layer was separated, desiccated with $Na_2SO_4$ anhydride and taken to dryness in vacuo, affording 26 g of crude extract.

REFERENCE EXAMPLE 3: Preparation of product (containing extract) and placebo for skin application.

Two creams with the composition shown in Table 1 below were manufactured according to the following procedure:

In an appropriated vessel, weight ingredients 6 to 14 and heat the mixture around 75°C.

In a vessel with enough capacity to contain the whole mixture, weight ingredients 1 to 5 and heat the mixture around 75°C.

While stirring the mixture, add the oil phase into the water phase.

Let the resulting mixture cool down until it reaches a temperature of around 30°C, add ingredients 15 and 16 and, while stirring let the mixture cool down until it reaches room temperature.

Add ingredient 17 and check pH (5.0-6.0). If pH is out of the range is adjusted with NaOH or citric acid

TABLE 1

| # | INGREDIENT | % (w/w) | |
|---|---|---|---|
| | | PRODUCT (with extract) | PLACEBO |
| 1 | Desionized Water % | Up to 100 | Up to 100 |
| 2 | Glycerin | 5 | 5.008 |
| 3 | Benzoic Acid | 0.7 | 0.7 |
| 4 | Sorbic Acid | 0.7 | 0.7 |
| 5 | Dehydroacetic Acid | 0.5 | 0.5 |
| 6 | Butyrospermun Parkii | 15 | 15 |
| 7 | Helianthus Annuus Oil | 5 | 5 |
| 8 | Cetearyl alcohol | 3 | 3 |
| 9 | Sodium Cetearyl | 2 | 2 |
| 10 | Decyl Oleate | 2 | 2 |
| 11 | Glyceryl Stearate | 1.5 | 1.5 |
| 12 | Cera Alba | 1 | 1 |
| 13 | Benzyl alcohol | 0.8 | 0.8 |
| 14 | Tocopheryl Acetate | 0.5 | 0.5 |
| 15 | Phenoxyethanol | 0.5 | 0.5 |
| 16 | Aspergillus Versicolor extract of example 2 | 0.008 | - |
| 17 | Parfum | 0.15 | 0.15 |

REFERENCE EXAMPLE 4: Antioxidant effect of *Aspegillus versicolor* by performing the DPPH (1,1-diphenyl-2-picrylhydrazyl) test

When a product capable of donating an hydrogen atom is contacted DPPH (1,1-diphenyl-2-picrylhydrazyl) DPPH is reduced with a consequent color loss and a loss in the absorbance which can be spectrophotometrically quantified.

The 150 µl of solutions of the products to be tested for antioxidant capacity were added in a 96 well plate together with 50 µl of a 0.1 mM DPPH solution and incubated during 30 minutes in the dark at room temperature. After the incubation time, antioxidant capacity was determined by comparing the absorbance at 517 nm of the product to be tested with absorbance at 517 nm of the control according to the formula below:

$$\% \text{ Antioxidant effect} = \left[1 - \frac{Sample\ absorbance}{Control\ absorbance}\right] \times 100$$

The above-mentioned test is used with four different aqueous solutions: water (control), 80µg/ml of *Aspergillus versicolor* extract of example 2 and 40µg/ml of *Aspergillus versicolor* extract of example 2 and the results are shown in Table 2.

TABLE 2

|  | $A_{517nm}$ (Mean ± SD) | Antioxidant effect (%) |
|---|---|---|
| DPPH (control) 0.1 mM | 0.134 ± 0.010 | - |
| Extract 40 μg/ml | 0.113 ± 0.004 | 16.0 |
| Extract 80 μg/ml | 0.105 ± 0.013 | 21.4 |

REFERENCE EXAMPLE 5: Cellular protection vs. oxidative stress induced by $H_2O_2$:

Cell viability after the addition of different product concentrations (50 μtg/ml, 250 μtg/ml, 500 μtg/ml, 1000 μg/ml) followed by incubation and addition of $H_2O_2$ (200 μg/ml), compared to basal control results.

The products to be tested were added to 96 well plates dissolving each product in 500 μl of methanol:DMSO (9:1). Once all products have been added the plates are left uncovered until the solvent evaporates.

A Carcinoma cell line A549 was cultured in RPMI 1640 medium (Sigma-Aldrich) supplemented with 5% FBS (Fetal Bovine Serum), 1% L-Glutamine and 1% penicillin/streptomycin. The cells were kept at 37 ± 2 °C in a wet atmosphere having 5 ± 1.5% $CO_2$.

The assay was done in duplicate and the mother solutions were prepared before each use. Cells were allowed to grow until 80% confluence was reached. Then, they were seeded to a density of $1 \times 10^4$ cells/ml in the 96 well plates containing the products to be tested and were incubated for 2 hours to obtain a correct adhesion. After said 2 hours $H_2O_2$ was added at a concentration of 200 μM and the plates were incubated during 48 hours at 37 ± 2 °C in a wet atmosphere having 5 ± 1.5% $CO_2$. Then, the supernatant liquid was removed from the wells which were washed twice with PBS (Phosphate buffered saline). Then, MTT (3-(4,5-dimethylthiazol-2-yl)-2,5-diphenyltetrazolium bromide) was added to the wells at a concentration of 5 mg/ml and the plate was incubated for 4 hours. Finally, 0.1 ml DMSO was added to each well agitating during 5 minutes. The absorbance of each well was read in a UV-Vis spectrophotometer at 550 nm with a reference wavelength of 620 nm.

Cell viability was determined by comparing the absorbance of the product to be tested with the absorbance of a control to which no $H_2O_2$ and no product had been added according to the formula below:

$$\% \text{ Cell viability} = \left[ \frac{Sample\ absorbance}{Control\ absorbance} \right] \text{x } 100$$

The results are shown in Table 3 show that the product has a significant antioxidant action, following a dose-dependent function. This product is able to protect the cells against oxidative stress induced by a powerful oxidizing agent ($H_2O_2$).

TABLE 3

| Cell viability (%) | | |
|---|---|---|
| Control | | 100 |
| $H_2O_2$ (200 μM) | Extract 0 μg/ml | 36 |
| | Extract 50 μg/ml | 56 |
| | Extract 250 μg/ml | 78 |
| | Extract 500 μg/ml | 99 |
| | Extract 1000 μg/ml | 100 |

REFERENCE EXAMPLE 6: Protection against $H_2O_2$-induced lipid peroxidation

Malondialdehyde (MDA) is a natural product of lipid peroxidation and the final product detected after the hydrolysis of lipid peroxides. It is considered as a good marker of the oxidative damage of biological membranes. The present example determines the production of MDA produced by Human Dermal Fibroblast exposed to $H_2O_2$ and the effect of the extract in said production.

A cell line of human dermic fibroblast was cultured in DMEM (Dulbecco's Modified Eagle Medium) with a glucose level supplemented with 10% FBS, 1% 2 mM L-Glutamine solution and 1% penicillin/streptomycin

The cells were maintained in an incubator at 37 $\pm$ 2 °C in a humidified atmosphere at 5 $\pm$ 1.5% $CO_2$ in culture flasks.

The assay was performed in duplicate and the stock solutions were prepared before each use.

The cells were allowed to grow until 80% confluence was reached. They were then seeded at a density of 1 x $10^4$ cells/ml in 96 well plates and incubated for 24 hours for proper adhesion. After this time, the medium was removed and the solution of product to be tested was added exposing cells to said solution for 48 hours. The tested solutions were: Culture media (basal control), 100 $\mu$M $H_2O_2$ (positive control) and solutions comprising 100 $\mu$M $H_2O_2$ and 80 $\mu$g/ml, 250 $\mu$g/ml, 500$\mu$mg/ml of and 1000 $\mu$g/ml of the extract of example 2 respectively.

After exposure, the supernatant liquid was removed from the wells which were washed twice with PBS. Then a solution comprising 0.5% Triton X100 in PBS was added to the wells to elicit cell lysis required for use TBARS Assay kit (Item n° 700870 Cayman Chemical). The incubation reaction was allowed to take place during 1 hour at 100 ° C followed by cooling the on ice for 10 minutes. The vials were centrifuged at 1600xg at 4 °C for 10 minutes. The samples and standards were placed in a 96 well plate and the absorbance was read at a wavelength of 530-540 nm in a UV-Vis spectrophotometer.

The concentration of MDA (in $\mu$M) was calculated from the absorbance (A) measurements using the following formula:

$$\text{MDA } (\mu M) = \left[ \frac{Absorbance\ of\ the\ sample - Intersection}{Slope} \right]$$

where the values "intersection" and "slope" are those of a previously determined MDA calibration line.

The results shown in Table 4 show that the product has a significant dose-dependent capacity to reduce the amount of MDA generated when used together with $H_2O_2$.

TABLE 4

|  |  | MDA ($\mu$M) | Decrease (%) |
|---|---|---|---|
| Control |  | 0 | - |
| $H_2O_2$ (500 $\mu$M) | Extract 0 $\mu$g/ml | 10.71 | 0 |
|  | Extract 80 $\mu$g/ml | 7.14 | 33.3 |
|  | Extract 250 $\mu$g/ml | 5.71 | 46.6 |
|  | Extract 500 $\mu$g/ml | 4.64 | 56.6 |
|  | Extract 1000 $\mu$g/ml | 3.92 | 63.3 |

REFERENCE EXAMPLE 7: Anti-inflammatory activity

Prostaglandin $E_2$ ($PGE_2$) is a product of arachidonic acid metabolism which does not exist in preformed form in cell deposits. When cells are stimulated by an inflammatory agent $PGE_2$ is synthesized *de novo* and released into the extracellular space. Thus quantification of $PGE_2$ is used to asses the anti-inflammatory activity products.

In the present example the anti-inflammatory activity of the extract of example 2 was determined by quantification of $PGE_2$ in cells stimulated by IL-1$\alpha$ using a kit for an enzyme-linked immunosorbent assay (ELISA). The kit used was Prostaglandin $E_2$ EIA kit Item n° 514010 from Cayman Chemical.

Prostaglandin $E_2$ assay is based on competition between the $PGE_2$ to be determined and a fixed amount of added $PGE_2$-acetylcholinesterase (AChE) conjugate ($PGE_2$ tracer) for a limited amount of $PGE_2$ monoclonal antibody. The amount of $PGE_2$ tracer able to bind the $PGE_2$ monoclonal antibody will be inversely proportional to the concentration of $PGE_2$ in the well. Both the antibody-$PGE_2$ complex and then antibody-$PGE_2$ tracer complex bind to polyclonal goat anti-mouse IgG previously bound to the well. Then the plate is washed to remove any unbound reagent and Ellman's reagent is added to determine the amount of AChE bound to the well. The determination is made by measuring colorimetrically the amount of a characteristic reaction product resulting from an enzymatic reaction catalyzed by AChE. Said reaction product absorbs at a wavelength of 412 nm. The intensity of this color, determined by UV-Vis spectrophotometry, is proportional to the amount of $PGE_2$ tracer bound free to the source, which is inversely proportional to the amount of $PGE_2$ present in the free well during incubation.

The results shown in Table 5 show that the product has a significant capacity to reduce the amount of $PGE_2$ thus confirming its anti-inflammatory properties.

TABLE 5

|  |  | PGE$_2$ (pg/ml) | PGE$_2$ Inhibition (%) |
|---|---|---|---|
| Control |  | 47.89 | - |
| IL-1$\alpha$ (5 pM) | Extract 0 $\mu$g/ml | 1308.81 | - |
|  | Extract 80 $\mu$g/ml | 5.56 | 99.6 |
|  | Cycloheximide 100 $\mu$g/ml | 31.85 | 97.6 |

REFERENCE EXAMPLE 8: Stimulation of production of intracellular ATP

Intracellular ATP production is a sign of correct mitochondrial function. It is known that oxidative processes have a negative impact on mitochondrial functions which translates into decreased levels of Intracellular ATP production.

The present example determines the increase in production of ATP by Human Dermal Fibroblasts exposed to the extract of example 2.

A cell line of human dermic fibroblast was cultured in a culture medium consisting of DMEM (Dulbecco's Modified Eagle Medium) with a glucose level supplemented with 10% FBS, 1% 2 mM L-Glutamine solution and 1% penicillin/streptomycin

The cells were maintained in an incubator at 37 $\pm$ 2 ° C in a humidified atmosphere at 5 $\pm$ 1.5% CO$_2$ in culture flasks.

The assay was performed in duplicate and the stock solutions were prepared before each use.

The cells were allowed to grow until 100% confluence was reached. They were then seeded at a density of 5 x 10$^4$ cells/ml in 24 well plates and incubated for 24 hours for proper adhesion. After this time, the medium was removed and the solution of product to be tested was added exposing cells to said solution for 24 hours.

After exposure, the supernatant liquid was removed from the wells which were washed twice with cold PBS. Then, the ATP buffer was added to elicit cell lysis required for the ATP analysis.

To achieve deproteination 500 $\mu$l of the samples were mixed with 100 pl of 4M cold perchloric acid and maintained in an ice bath during 5 minutes. The samples were centrifuged at 4°C during 2 minutes and supernatants were collected. Then, a 2M KOH cold aqueous solution was added. The samples were centrifuged at 4°C during 15 minutes and supernatants were collected.

To perform the ATP determination 50 ml of standard mixture were added to each well in the plate followed by 50 ml of culture medium or a solution of the extract of example 2 in culture medium. Then the mixtures were incubated at room temperature during 30 minutes in the dark. After the incubation period the absorbance of each well in plate was recorded in a UV-Visible spectrophotometer at 570 nm.

Using a previously obtained standard calibration curve the amount of ATP (in nmol/$\mu$l) was calculated by interpolation in the curve.

The results shown in Table 6 show that the extract increases the production of intracellular ATP.

TABLE 6

|  | A$_{570nm}$ (Mean $\pm$ SD) | ATP ($\mu$M) | ATP Increase (%) |
|---|---|---|---|
| Basal control | 1.29 $\pm$0.12 | 6.56 | - |
| Extract 80$\mu$g/ml | 1.49 $\pm$0.14 | 7.72 | 27.4 |

REFERENCE EXAMPLE 9: Determination of antipollutant effect of pretreatment against the effect of CdCl$_2$

CdCl$_2$ is a chemical compound used for the determination of the environmental contamination effect on skin.

Human Dermal Fibroblasts were incubated in a 96 well plates until reach 80% of confluence. Control or extract of example 2 (5 $\mu$g/ml in DMEM) were added to the well and incubated for 24 hours, and then CdCl$_2$ was added at different concentrations (2 $\mu$M, 4 $\mu$M, 6 $\mu$M, 8 $\mu$M and 10 $\mu$M), as a challenge agent and incubated 24 additional hours. After the incubation period, cell viability was measured by MTT assay as described in Example 5.

The results shown in Table 7 show that the extract protects cells from CdCl$_2$ induction.

TABLE 7

|  | Cell viability (%) | |
|---|---|---|
| CHALLENGE | Without extract | With 5 $\mu$g /ml of extract |
| $CdCl_2$ 0$\mu$M | 100 | 100 |
| $CdCl_2$ 2$\mu$M | 83.5 | 100 |
| $CdCl_2$ 4$\mu$M | 75.8 | 100 |
| $CdCl_2$ 6$\mu$M | 72.6 | 100 |
| $CdCl_2$ 8 $\mu$M | 63.6 | 100 |
| $CdCl_2$ 10$\mu$M | 56.1 | 100 |

REFERENCE EXAMPLE 10: Determination of antipollutant effect of post-treatment against the effect of $CdCl_2$

Human Dermal Fibroblasts were incubated in a 96 well plates until reach 80% of confluence. $CdCl_2$ was added at different concentrations (2 $\mu$M, 4 $\mu$M, 6 $\mu$M, 8 $\mu$M and 10 $\mu$M) in different wells and incubated for 24 hours. After the incubation period extract of example 2 (5 $\mu$g/ml in DMEM) was added to the wells and incubated for 24 additional hours, and then, cell viability was measured by MTT assay as described in Example 5.

The results shown in Table 8 show that the extract treats cells from $CdCl_2$ induction.

TABLE 8

|  | Cell viability (%) | |
|---|---|---|
| CHALLENGE | Without extract | With 5$\mu$g/ml extract |
| $CdCl_2$ 0$\mu$M | 100 | 100 |
| $CdCl_2$ 2$\mu$M | 83.5 | 100 |
| $CdCl_2$ 4$\mu$M | 75.8 | 98.5 |
| $CdCl_2$ 6$\mu$M | 72.6 | 78.4 |
| $CdCl_2$ 8 $\mu$M | 63.6 | 77.7 |
| $CdCl_2$ 10$\mu$M | 56.1 | 77.1 |

REFERENCE EXAMPLE 11: Double-blind, randomized, placebo-controlled test on the efficacy of a cream comprising the extract of example 3

Selection of test subjects

The test subjects were healthy females having Caucasian skin type and an age ranging from 30 to 70 and having a healthy skin area (free of psoriasis, eczema, erythema, edema, scars, wounds or lesions). The subjects did not have hypersensitivity or skin allergy to cosmetics products and should not have used in the area of the skin used during the test any product or make up. The subject should also not have used, in the 2 weeks preceding the study, firming products or products with an anti-wrinkle or anti-ageing action, or A.H.A-based cosmetics (fruit acids, derivates) or retinol-based cosmetics. The subject should also not have received skin-treating injections such as Botox, collagen, hyaluronic acid and the like.

The days on which the measurements are to be taken, the application of any other cosmetic product and/or make up, including the usual cleanser and the tested products to the studied areas is proscribed (only face cleaned with water is accepted). During the study, the application of any other cosmetic product to the studied areas is proscribed (only the usual cleanser and make-up for the lips, eyes and blusher are accepted). It is also proscribed for the subjects to expose themselves to artificial UV light and/or to the sun during the study.

Products used and application of the products

The Table 9 below shows the application of the test products to the subjects.

TABLE 9

| Product | Application area | Frequency of application | Application duration |
|---|---|---|---|
| PRODUCT containing *Aspergillus versicolor* of example 3 | Hemiface (right) | Twice daily (morning and evening) | 28 days |
| PLACEBO of example 3 | Hemiface (left) | Twice daily (morning and evening) | 28 days |

The products are applied by the subjects themselves, twice daily (morning and evening) according to a pre-established randomized pattern, half-face and neck, during 28 days. Quantities of application should correspond to normal conditions of use. Subjects have to wash their hands between applications of each product.

This is a comparative study in which the results obtained at one treated area by one of the products are compared with those obtained at another treated area with a placebo. The subjects serve as their own reference and results obtained at various assessment times are compared with those obtained at T0. The selection of the areas to be treated (right or left side of face and neck) with each one of the products is determined at random for each subject. An algorithm designed for this purpose carries out this randomization.

Quantification of Glycation by Autofluorescence Spectroscopy

The quantification of glycation end-products was made with the help of a specific device called AGE Reader. The AGE Reader measures tissue accumulation of Advanced Glycation End-products (AGEs) by means of fluorescence techniques (skin autofluorescence (skin AF)). The AGE Reader has a light source, which illuminates the skin. This light will excite fluorescent moieties in the tissue, which will emit light of a different wavelength. In the used wavelength band ($\lambda$p = 375nm) the major contribution in fluorescence comes from fluorescent AGEs linked mostly to collagen, but also to other proteins and lipids. The emitted light is detected using a spectrometer.

By using specific technical adaptations including selection of specific wavelength, modulated or pulsed light sources ($\lambda$p = 460nm + $\lambda$p = 880nm), a more selective discrimination of specific AGEs is obtained.

Age is the most important factor in determining the levels of advanced glycation end-products (AGE) in tissue. AGEs value as measured by the AGE Reader is also correlated with the subject's age. Reference Values for AGEs values are based on measurements in 456 Caucasian subjects (smokers and non-smokers): [Lutgers et al, Diabetes Care 2006, y Koetsier et al, Diabetes Technology & Therapeutics., 2010; 12 (5): 399-403].

To carry out the measurements with the AGE reader the subject is requested to rest its forearm or cheek over the reader's silicone platform. If the measurement is carried out in the face, then the subject places one cheek flat on the silicone platform while wearing a mobcap.

The AGEs is used to make measurements in the forearm and face at D0 to validate the face results, but the measurements at day 28 will be taken only on the subjects' face. The measurements are performed on both cheeks. The site of the instrumental measurements and their location at the different points of the kinetics should be strictly the most reproducible.

The data analysis is carried out on the device, which provides not only a numerical value of the measurement on a scale of 0 to 5 (wherein lower values indicate lower amount of AGEs) but also a graphical representation showing the position of the measured value on a graph depicting correlation of the values with mean values of subjects having the same age. The AGEs index values of individuals between 30 and 70 are normally within the range of 1 to 3 and increase with age. A decrease in AGE index indicates a reduction of AGEs which translates in an increase in skin elasticity. It is well known that elastic fibers play a role in maintaining skin elasticity. An amino acids residue such as lysine and arginine are susceptible to glycation. Glycated lysine and arginine form crosslinks between fibers leads to a loss of elastic fibers mobility.

For each point of the skin assessed 3 measurements are taken to reduce the dispersion of the values of the Glycation Autofluorescence Index. The average and standard deviation of these measurements is calculated from theses measurements.

The following tables present the means and the standard deviations on the raw values and the percentages of variation for the Glycation Autofluorescence Index measured on the cheeks at D0 D28, for the sides treated by the Product and by the Placebo, as well as the corresponding statistical results for the evolution in time (Student, two-tailed for paired groups at 5%, after checking the normality of the distributions by a Shapiro-Wilk test and Kolmogorov-Smirnov; if the result is statistically significant the p-value is underlined).

TABLE 10

| Formulation | % of change |
|---|---|
| Product containing *Aspergillus versicolor* | -11.9 |
| Placebo | 5.5 |

16 subjects out of 19 subjects treated with Product (i.e. 84.2% of the panel) showed a reduction in AGE the AGE index indicating a decrease in AGEs.

4 subjects out of 19 subjects treated with Placebo (i.e. 21.1% of the panel) showed a reduction in AGE the AGE index indicating a decrease in AGEs.

The Table 11 below summarizes the results of the statistical analysis of the data obtained in the test.

TABLE 11

|  | a | p |
|---|---|---|
| Product @ day 0 vs. Placebo @ day 0 | 0.6500 | p>0.05 NS |
| Product @ day 0 vs. Product @ day 28 | 0.0307 | p<0.05 * |
| Placebo @ day 0 vs. Placebo @ day 28 | 0.2295 | p>0.05 NS |
| Placebo @ day 28 vs. Product @ day 28 | 0.0084 | P<0.01 ** |
| Placebo @ day 28 - Placebo @ day 0 vs. Product @ day 28 - Product @ day 0 | 0.0012 | p<0.005 *** |

A significant variation in the studied parameter is observed after 28 days of application of Product.

No significant variation in the studied parameter is observed after 28 days of application of Placebo.

A significant difference of evolution is observed between studied sides, 28 days after application of products A and B on the studied parameters.

Quantification of Antioxidants by Autofluorescence Spectroscopy

The quantification of antioxidants was made with the help of a specific handheld device called Biozoom. Biozoom measures carotenoids present in the skin by means of fluorescence techniques (skin autofluorescence). Biozoom has light sources which illuminate the skin. Those lights will excite β-carotenes in the skin tissues causing them to emit light of a different wavelength. The wavelength band used for emission is 440-480 nm and the wavelength band sampled for fluorescence was 400-500 nm.

As the antioxidants form protective chains, which allow them to regenerate after neutralizing free radicals, the analysis of one main antioxidant substance is sufficient to yield information regarding the antioxidant status as a whole. Using electro paramagnetic resonance spectroscopy, it has been demonstrated that, for example, carotenoids represent indicator marker substances for the overall antioxidant status of the epidermis. In the case of Biozoom, several studies carried out at la Charité Hospital in Berlin have proven that Biozoom is as reliable as conventional laboratory spectroscopy techniques for the measurement of skin carotenoids.

To carry out the measurements with Biozoom the subject is requested to sit on a photographic acquisition bench wearing a mobcap. This bench allows us to keep the face in the same position and the handheld Biozoom is used for the measurement. The measurements are performed on both cheeks. The Biozoom is placed over the measurement point without compressing the skin while the measurement takes place. The Biozoom device is connected to a PC via a USB cable. The data from the device are transferred to the Biozoom's servers where they are processed to return a "carotenoids autofluorescence index" displayed on the screen of the device on a scale from 0 to 10. At each location 3 measurements are taken to reduce the dispersion of the values.

The data summarized Table 12 below contain the means and the standard deviations calculated from the Carotenoids Autofluorescence Scores obtained with Biozoom measurements as indicated above.

The data were collected at day 0 and day 28 after treatment with products A and B.

TABLE 12

| Formulation | % of change |
|---|---|
| Product | 12.1 |

(continued)

| Formulation | % of change |
|---|---|
| Placebo | -0.7 |

The Table 13 below summarizes the results of the statistical analysis of the data obtained in the test.

TABLE 13

| | a | p |
|---|---|---|
| Product @ day 0 vs. Placebo @ day 0 | 0.4551 | p>0.05 NS |
| Product @ day 0 vs. Product @ day 28 | 0.0166 | p<0.05 * |
| Placebo @ day 0 vs. Placebo @ day 28 | 0.9999 | p>0.05 NS |
| Placebo @ day 28 vs. Product @ day 28 | 0.0218 | p<0.05 * |
| Placebo @ day 28 - Placebo @ day 0 vs. Product @ day 28 - Product @ day 0 | 0.0316 | p<0.005 *** |

A significant variation in the studied parameter is observed after 28 days of application of Product.

No significant variation in the studied parameter is observed after 28 days of application of Placebo.

A significant difference of evolution is observed between studied sides, 28 days after application of Product and Placebo on the studied parameters.

The present application also relates to:

[1] A microorganism from the species Aspergillus versicolor deposited in the Spanish Type Culture Collection (CECT) with accession number CECT 20952.

[2] A biologically pure culture of a microorganism according to [1].

[3] A broth obtainable by aerobic incubation of an aqueous composition comprising a source of carbon, a source of $K^+$, a source of $Mg^{2+}$, a source of $Na^+$, a source of $Cl^-$, a source of $SO_4^{2-}$, a source of $PO_4^{3-}$ in the presence of a microorganism according [1] at a pH between 5 and 7.

[4] An extract obtainable by separating the microorganism from the broth according to [3] extracting the resulting solution with an organic solvent such as ethyl acetate, recovering the organic layer and removing the organic solvent from said organic layer.

[5] A cosmetic composition comprising an effective cosmetic quantity of the extract the extract as defined in [4] and at least one cosmetically acceptable excipient, adjuvant and/or ingredient.

[6] Use of the extract of [4] or the cosmetic composition of [5] for the cosmetic, non-therapeutic treatment and/or care of the skin.

[7] Use of the extract of [4] for the manufacture of a cosmetic composition for treating the skin.

[8] Use according to any one of [6] or [7] wherein the treatment is capable of one or more of the following actions: a) reducing the presence of advanced glycation end-products, b) protecting the skin against oxidative stress, c) increasing the synthesis of intracellular ATP, d) increase skin density, e) increase skin thickness, f) protecting the skin from the effects of contamination, g) protecting the skin from the effects UVb radiation.

**Claims**

1. A microorganism from the species *Aspergillus versicolor* deposited in the Spanish Type Culture Collection (CECT) with accession number CECT 20952.

2. A biologically pure culture of a microorganism according to claim 1.

3. A broth obtainable by aerobic incubation of an aqueous composition comprising a source of carbon, a source of $K^+$, a source of $Mg^{2+}$, a source of $Na^+$, a source of $Cl^-$, a source of $SO_4^{2-}$, a source of $PO_4^{3-}$ in the presence of a microorganism according to claims 1 at a pH between 5 and 7.

4. An extract obtainable by separating the microorganism from the broth according to claim 3, extracting the resulting solution with an organic solvent such as ethyl acetate, recovering the organic layer and removing the organic solvent from said organic layer.

Europäisches Patentamt

European Patent Office

Office européen des brevets

## EUROPEAN SEARCH REPORT

Application Number

EP 16 38 2621

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ARAI T ET AL: "Ultraviolet ray absorbing substances produced by Aspergillus microorganisms - used as industrial preservatives and cosmetic additives", WPI / 2017 CLARIVATE ANALYTICS, vol. 1995, no. 7, 29 November 1994 (1994-11-29), XP002768085, * the whole document * | 1-4 | INV. C12N1/14 C12P1/02 A61K8/99 A61Q19/00 A61Q19/08 ADD. C12R1/66 |
| A | -& JP H06 329576 A (KAIYO BIO TECH LAB) 29 November 1994 (1994-11-29) * the whole document * | 1-4 | |
| A | WU Z. ET AL: "Antioxidative phenolic compounds from a marine-derived fungus Aspergillus versicolor", TETRAHEDRON, vol. 72, no. 1, 19 October 2015 (2015-10-19), pages 50-57, XP029345003, ISSN: 0040-4020, DOI: 10.1016/J.TET.2015.10.038 * the whole document * | 1-4 | |
| A | CHEN Y. ET AL: "Structure and antioxidant activity of an extracellular polysaccharide from coral-associated fungus,LCJ-5-4", CARBOHYDRATE POLYMERS, vol. 87, no. 1, 22 July 2011 (2011-07-22), pages 218-226, XP028316758, ISSN: 0144-8617, DOI: 10.1016/J.CARBPOL.2011.07.042 [retrieved on 2011-07-30] * the whole document * | 1-4 | |

TECHNICAL FIELDS SEARCHED (IPC)

C12N
C12R
A61K
C12P
A61Q

-/--

The present search report has been drawn up for all claims

| Place of search | Date of completion of the search | Examiner |
|---|---|---|
| The Hague | 26 April 2017 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 16 38 2621

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HANADA K ET AL: "New decalin derivatives isolated from Aspergillus versicolor F5015 - prevent nerve cell decrease and are useful for treating dementia", WPI / 2017 CLARIVATE ANALYTICS, vol. 1993, no. 11, 9 February 1993 (1993-02-09), XP002767618, * the whole document * | 1-4 | |
| A | -& JP H05 32656 A (TAISHO PHARMA CO LTD) 9 February 1993 (1993-02-09) * the whole document * ----- | 1-4 | |
| A | DUARTE K. ET AL: "Analytical techniques for discovery of bioactive compounds from marine fungi", TRENDS IN ANALYTICAL CHEMISTRY, vol. 34, 23 January 2012 (2012-01-23), pages 97-110, XP028472046, ISSN: 0165-9936, DOI: 10.1016/J.TRAC.2011.10.014 * the whole document * ----- | 1-4 | |

TECHNICAL FIELDS
SEARCHED (IPC)

The present search report has been drawn up for all claims

| Place of search | Date of completion of the search | Examiner |
|---|---|---|
| The Hague | 26 April 2017 | van de Kamp, Mart |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

page 2 of 2

## ANNEX TO THE EUROPEAN SEARCH REPORT
## ON EUROPEAN PATENT APPLICATION NO.

EP 16 38 2621

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2017

| Patent document cited in search report | | Publication date | Patent family member(s) | Publication date |
|---|---|---|---|---|
| JP H06329576 | A | 29-11-1994 | NONE | |
| JP H0532656 | A | 09-02-1993 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Harry's Cosmeti-cology. Longman House, 1982
- **LUTGERS et al.** *Diabetes Care,* 2006
- **KOETSIER et al.** *Diabetes Technology & Therapeutics,* 2010, vol. 12 (5), 399-403